(19) Europäisches Patentamt / European Patent Office / Office européen des brevets

(11) **EP 3 922 236 A1**

(12) **EUROPEAN PATENT APPLICATION**

(43) Date of publication:
**15.12.2021   Bulletin 2021/50**

(51) Int Cl.:
*A61K 8/35* *(2006.01)*    *A61K 8/37* *(2006.01)*
*A61K 8/40* *(2006.01)*    *A61K 8/60* *(2006.01)*
*A61K 8/64* *(2006.01)*    *A61Q 17/04* *(2006.01)*
*A61Q 19/02* *(2006.01)*    *A61Q 19/08* *(2006.01)*

(21) Application number: **21177818.8**

(22) Date of filing: **04.06.2021**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:   **08.06.2020   IT 202000013570**

(71) Applicant: **Professional Dietetics S.p.A.**
**20129 Milan (IT)**

(72) Inventor: **GIORGETTI, Paolo**
**20129 MILANO (IT)**

(74) Representative: **Bianchetti & Minoja SRL**
**Via Plinio, 63**
**20129 Milano (IT)**

(54) **COMPOSITIONS FOR TOPICAL TREATMENT OF ACQUIRED MELANOSIS OF THE SKIN**

(57)   Disclosed are topical formulations comprising oligopeptide-68, encapsulated in vesicular structures selected from liposomes, niosomes, transfersomes and ethosomes, N-acetyl glucosamine and at least one UVA sunscreen, optionnaly combined with one or more UVB sunscreens, useful for the treatment of cases of acquired melanosis, post-inflammatory melanosis, UV-induced melanosis and photoaging which are resistant to treatment with common depigmenting substances.

**EP 3 922 236 A1**

**Description**

**Field of invention**

The present invention relates to topical formulations comprising oligopeptides with an MITF-inhibiting action, N-acetyl glucosamine and at least one UVA sunscreen, combined with a skin absorption carrier, useful for the treatment of cases of acquired melanosis, post-inflammatory melanosis, UV-induced melanosis and photoaging which are resistant to treatment with common depigmenting substances.

**Background to the invention**

Melanins, i.e. pigments that protect the skin, and therefore the body, against the action of UV radiation, are a heterogeneous class of molecules produced by cells located in the epidermis called melanocytes. Melanins are divided into eumelanins (EM), i.e. dark melanins that give the skin its particular tanned appearance after exposure to the sun or other UV sources, and pheomelanins (FM), i.e. lighter melanins synthesised in smaller amounts, depending on genotype.

Melanins are highly complex molecular polymers which are synthesised in the melanocyte by the action of numerous enzymes, especially L-tyrosine. Tyrosinase, the first enzyme responsible for melanin biosynthesis, triggers the biochemical cascade that leads to conversion of tyrosine into DOPA, the substrate which, after the involvement of other enzymes, gives rise to melanin synthesis. Said enzyme is therefore the main target of substances with lightening activity, which are useful, for example, to induce lightening of the melanin accumulations responsible for lesions such as melasma.

Melasma is a brown facial pigmentation that is very common in young women, affecting the forehead, cheekbones and "moustache" area.

The cause has not been fully clarified, but genetic predisposition, sun exposure and stimulus from female hormones are certainly involved. The condition is chronic, and tends to recur on every exposure to the sun.

The available treatments can be medical or surgical, depending on the characteristics of the lesion. The former consist of surface peeling, i.e. application to the skin surface of substances with exfoliating and keratolytic activity able to remove the more superficial pigmented cells, combined with home treatment based on depigmenting products and sunscreens.

This type of treatment regimen is very effective against the superficial forms of the condition, whereas in the dermoepidermal variety, Q-switched laser or laser refreshing treatments can be attempted.

Post-inflammatory pigmentations are lesions caused by accumulation of melanic pigment induced by an inflammatory stimulus. They are generally found in areas exposed to light, and their appearance is connected with exposure to UV radiation and physical irritant stimuli (such as lamps, sun exposure and burns). Radiation is a pro-inflammatory stimulus that generates the production of cytokines and inflammatory mediators which, by acting on the melanocyte, induce it to increase synthesis of melanic pigment. The biochemical action mechanism of this phenomenon is basically increased transcription of melanogenesis enzymes (tyrosinase and TRP up-regulation).

Dermocosmetological science has identified various substances of natural, extracted or synthetic origin able to perform an inhibitory action against melanin synthesis in the melanocyte. Said substances interfere with melanin synthesis. The main action mechanisms underlying the activity of substances with melanic depigmenting action are:

- modulation of gene expression and transcription of melanogenesis enzymes;
- interference with the catalytic activity of the enzyme pool responsible for melanogenesis;
- promotion of intra-melanocyte synthesis of glutathionyl-DOPA (pheomelanins);
- inhibition of melanosome transfer;
- stabilisation of melanocyte mitochondria;
- inhibition of glycosidation of pro-tyrosinase to tyrosinase;
- inhibition of release of inflammatory mediators and endothelin;
- chemical or physical depletion of melanin accumulation in the epidermis;
- inhibition of Microphthalmia-associated Transcription Factor (MITF).

The latter activity, which was only investigated recently, plays a key role in modulating gene expression of tyrosine enzymes in the melanocyte, and in the differentiation thereof; MITF (Microphthalmia-associated Transcription Factor) is a peptide factor involved in differentiation of melanocytes and modulation of gene transcription of tyrosine enzymes. Some currently available oligopeptides have a signalling function able to induce, with high specificity, down-regulation in the gene expression of enzymes associated with tyrosinase, leading to significant biosynthesis of eumelanins.

The cosmetological approach to melanic pigmentation is often insufficient to produce a significant aesthetic correction, because of the biochemical complexity of the melanin synthesis process and the difficulty of conveying the active substances to the melanocyte, located in the deep epidermis. Combinations of substances that act at different stages of melanin synthesis and in melanocyte gene expression are more effective, but often fail to meet expectations in cases where only hydroquinone, a substance prohibited for cosmetic use due to its proven toxicity towards the melanocyte, so far seems to hold the record in terms of depigmenting efficacy (exogenous ochronosis; Menke HE, et al. Ned Tijdschr Geneesk 1992; 4: 136; Bruce S et al., J. Am. Acad. Dermatol. 1986 August; 15 (2 Part 2): 357-61; Findlay GH,. S. Afr. Med. J. 1980; 57: 187).

It would therefore be particularly desirable to have a combination of substances able to bring about a significant improvement in acquired hypermelanoses of various etiologies and maintain the result over time, without inducing the adverse or sensitising events typical of phenolic substances such as hydroquinone, even when used for long periods.

### Description of the invention

It has now been discovered that the combination of an oligopeptide with inhibitory action against MITF (Microphthalmia-Inhibiting Transforming Factor), N-acetyl glucosamine and at least one UVA sunscreen, applied topically, induces effective, lasting lightening of melanic pigmentation resistant to treatment with known lightening agents. The combination is particularly useful for the treatment of acquired hypermelanoses such as melasma, chloasma, UV-induced melanosis, inflammatory melanosis, and melanoses of various etiologies.

The subject of the invention is therefore topical formulations comprising oligopeptide 68 (CAS 1206525 47 4) with an MITF-inhibiting action, N-acetyl glucosamine, and at least one UVA sunscreen.

Oligopeptides with an MITF-inhibiting action are involved in modulating the gene transcription of tyrosinase enzymes, while N-acetyl glucosamine (NAG) is an aminosugar able to inhibit the glycosidase-dependent conversion process of pro-tyrosinase to tyrosinase.

Avobenzone (INCI: Butyl methoxydibenzoylmethane), octocrylene and mixtures thereof are preferably used as UVA sunscreens.

The formulations can also contain substances with a sunscreen action on the UVB component to enhance the sun-protection action and reach the indicated SPF value of not less than 15. Examples of UVB sunscreens comprise cinnamic acid derivatives (INCI: EthylHexyl Methoxycinnamate), salicylic acid derivatives (INCI: EthylhexylSalicylate) and Benzophenones (INCI: Benzophenone-3, Benzophenone-4).

The percentages by weight of the ingredients according to the invention can fall within a wide range, determinable by the skilled person using routine methods. Broadly speaking, however, the weight percentage of the oligopeptide can range from 0.01 to 10%, the percentage of N-acetylglucosamine from 0.01 to 10% of the total weight of the formulation, and the percentage of each UVA and UVB sunscreen can range from 2.5 to 12.5% by weight of the formulation, more preferably between 5 and 10%. The sun protection, measured on the DIN SPF scale, should be not less than 15, and guarantee a UVA protection efficacy according to the equation SP-UVA/SPF>1/3.

The compositions according to the invention will also preferably contain absorption promoters such as non-ionic surfactants like polysorbates (e.g. polysorbate 80), fatty acids or PEGylated derivatives thereof (e.g. PEG-40 hydrogenated castor oil), propylene glycol, anionic surfactants and combinations thereof. Oleic acid is a particularly preferred absorption promoter.

The peptide is encapsulated by known techniques in bilayer structures such as liposomes, transfersomes, ethosomes and niosomes. The preferred solution is encapsulation of the peptide in liposomes.

The formulations according to the invention can take the form of O/W emulsions, W/O emulsions, oils; or make-up bases (foundations, concealers or BB creams).

The formulations according to the invention can also contain other known depigmenting agents, selected, for example, from diacetyl boldine, azelaic acid, dipotassium azeloyl diglycinate, sodium ascorbyl phosphate, ethyl ascorbic acid, ascorbyl hyaluronate, soybean oil and arbutin, typically in amounts ranging from 0.01 to 10% by weight of the formulation.

The compositions according to the invention are more effective than hydroquinone in reducing tyrosinase expression, confirming that said composition can be successfully used in the treatment of acquired melanic pigmentation, especially in the case of tyrosinase hyperexpression associated with hyperoestrogenic activity. No less importantly, said composition can replace the use of hydroquinone, the toxicity whereof to the melanocyte is extensively documented, especially if applied at concentrations exceeding 3% and for long periods (exogenous ochronosis) and used during pregnancy (mask of pregnancy). The test conducted, reported below, demonstrates that the preparation containing said composition is superior in terms of absolute efficacy in inhibiting gene expression of tyrosinase in human melanoma cells, and maintaining higher cell viability than hydroquinone after application.

The example below illustrates the invention in greater detail.

*Example 1 ((O/W emulsion)*

| A | PEG-100 Stearate and Glyceryl Stearate | 2,50000 |
|---|---|---|
| A | Potassium cetyl phosphate. | 2,00000 |
| A | Ascorbyl Palmitate | 0,05000 |
| A | Tocopheryl acetate | 0,20000 |
| A | Caprylic/Capric Triglyceride | 1,00000 |
| A | Oleic acid | 0,04000 |
| A | Octyldodecanol | 23,0000 |
| A | Hydrogenated Vegetable Oil | 4,00000 |
| A | Cetyl Ethylhexanoate | 1,00000 |
| A | Ethylhexyl Palmitate | 1,00000 |
| A | Ethylhexyl Methoxycinnamate | 7,00000 |
| A | Avobenzone | 3,00000 |
| A | Octocrylene | 3,00000 |
| A | Diacetyl Boldine | 0,20000 |
| A | Phenoxyethanol (and) Benzoic Acid (and) Dehydroacetic Acid | 0,70000 |
| A | Phenoxyethanol (and) Ethylhexylglycerin (and) Octenidine HCl | 0,70000 |
| A | Dimethicone | 6,00000 |
| B | Aqua (water) | q.s. to 100 g |
| B | Sodium Hyaluronate | 0,40000 |
| B | Xanthan gum | 0,250000 |
| C | Acetylglucosamine | 0,40000 |
| C | Water (and) Butylene Glycol (and) Hydrogenated Lecithin (and) Sodium Oleate (and) Oligopeptide-68 (and) Disodium EDTA | 0,20000 |
| C | Ceramide NP; Ceramide AP; Ceramide EOP; Phytosphingosine; Cholesterol; Sodium Lauroyl Lactylate; Carbomer; Xanthan Gum | 4,00000 |
| D | Citric acid | q.s. to 4.3<pH<4.8 |

*Preparation method*

Prepare Phases A, B and C separately. Heat Phases A and B to 75°C and add Phase A to Phase B in successive volumes under vigorous mechanical stirring for 5 minutes until homogeneous. Mix for 5 minutes with a Silverson turboemulsifier on setting 3/4 for 5 minutes. Start cooling under mechanical stirring. Add Phase C at t<30°C, continuing stirring, and then mix again for a further 3 minutes. Add Phase D under stirring, and finally adjust pH to between 4.3 and 4.8 with a 50% sol. of citric acid.

*Example 2*

**Tyrosine expression reduction activity**

Purpose of test

The purpose of this test is to investigate and quantify changes in melanin synthesis in highly pigmented human melanoma cells treated with the test substance, compared with the untreated cells, evaluating the gene expression levels of tyrosinase, the enzyme mainly involved in converting tyrosine to melanin.

Real-time PCR is a quantitative method that allows the presence of a specific messenger RNA that encodes for tyrosinase, synthesised by the cells under specific conditions, to be amplified and analysed. The reduction in messenger RNA following stimulation is a strong indicator of the efficacy of the product in reducing synthesis of tyrosinase and, indirectly, of melatonin. Hydroquinone, a known depigmenting agent, was used as positive control.

### Cell model

The *in vitro* experimental model consists of human melanoma cell cultures (MNT1), namely cells with a fibroblastoid morphology that produce melanin.

### Treatment and exposure

The cells were seeded in 6-well plates for 24 hours at 100000 cells/well. Fresh medium was added with the test product at two different concentrations (0.2 and 0.1 mg/ml), selected on the basis of a preliminary cytotoxicity test. The untreated cells were used as negative control, and cells treated with 5 $\mu$g/ml of hydroquinone were used as positive control. Each sample was tested in triplicate.

After 72 hours' exposure, the total RNA is purified from the cells, using an automatic extractor (MaxwellrM RCS system), according to the manufacturer's instructions.

The RNA was resuspended in 50 $\mu$l of sterile water, and its concentration was determined spectrophotometrically. 1000 ng of total RNA was reverse transcribed to cDNA and diluted to conduct the Real-Time gene expression analysis.

### MTT cell viability assay

At the end of the incubation, cell viability is evaluated by incubating the cells for 2 hours with MTT solution (1000 $\mu$l per well). The precipitated formazan is then extracted with isopropanol (2000 $\mu$l per well) and quantified spectrophotometrically at 570 nm. The plate is shaken on a rotary plate, ensuring that all the crystals have been dissolved by the cells and formed a homogeneous solution. The absorbance is measured on a microplate reader (Tecan Infinite F200), subtracting the background at 650 nm.

### RealTime-PCR analysis of gene expression profile

The changes in the gene expression profile were analysed with the RealTime PCR technique, using the SYBR Green assay.

The specific primers available on the market were obtained from Biorad. The tyrosinase primer sequence was designed in the exon.

The SYBR Green fluorescence signal increases in direct proportion to the amount of PCR product in the reaction. By recording the amounts of fluorescence emitted in each cycle, the PCR can be monitored during the exponential phase, wherein the first significant increase in the amount of PCR product is correlated with the initial amount of the target sequence.

The SYBR Green fluorescence signal increases in direct proportion to the amount of PCR product in the reaction. By recording the amounts of fluorescence emitted in each cycle, the PCR can be monitored during the exponential phase, wherein the first significant increase in the amount of PCR product is correlated with the initial amount of the mould sequence.

### Expression of results

The result is expressed as percentage cell viability according to the formula % **cell viability** = [OD (570 nm-650 nm) product tested/mean OD (570 nm-650 nm) blank] x 100.

The changes in the gene expression profile were measured by the comparative Ct method ($\Delta\Delta C_t$ method). The data were standardised relative to the expression levels of beta-actin, as control gene. For the quantitative analysis, the difference between the Cr value of the target (tyrosinase) and that of the control gene (actin) was calculated, to obtain the value of $\Delta\Delta C_t$

$$\Delta\Delta C_t = Ct\ (target) - Ct\ (control)$$

Said value was calculated for each sample to be quantified.

The untreated sample (calibrator) was used as reference value for all the comparisons conducted.

$\Delta\Delta C_t$ was calculated by calculating the difference between the $\Delta C_t$ of each sample and the $\Delta C_t$ of the untreated control (calibrator)

$$\Delta\Delta C_t = \Delta C_t \text{ sample 1 - } \Delta C_t \text{ untreated control (calibrator)}.$$

$\Delta\Delta C_t$ was then used to determine the Fold Change values:

**Fold change = 2** $^{\Delta\Delta Ct}$

The change in the gene expression profile was measured with the comparative $C_t$ method ($\Delta\Delta C_t$ method).

The data of the sample were standardised to the expression level of actin as constitutive gene.

The difference between the Ct value of the target (tyrosinase) and the Ct of the actin gene was then evaluated.

$$\Delta C_t = \Delta C_t \text{ (target) - } \Delta C_t \text{ (constitutive)}.$$

$\Delta C_t$ was calculated for each sample.

The untreated sample was considered as reference (calibrator) and used for each comparison. $\Delta\Delta C_t$ was evaluated as the difference between the $\Delta C_t$ of each sample and the $\Delta C_t$ of the untreated sample (calibrator).

$$\Delta\Delta C_t = \Delta C_t \text{ sample 1 - } \Delta C_t \text{ untreated sample (calibrator)}$$

$\Delta\Delta C_t$ was used to calculate the **Fold Change values**

**Fold Change = 2-**$_{\Delta\Delta Ct}$

**Acceptance criteria of method**

For the positive control
Fold change <0.8 together with a P value <0.05 relative to the untreated cells
For the positive control
Fold change <0.8 together with a P value <0.05 relative to the untreated sample

**Interpretation of results**

A fold change <0.8 together with a P value < 0.05 relative to the values of the untreated cells indicates modulation of the gene analysed.

Said value is compared with the untreated cells to indicate the activity of the sample.

**RESULTS**

*Acceptance requirements of test*

|  | Value | Limits | Result/ |
|---|---|---|---|
| PC: fold change/percentage decrease | 0.701 | :S:0.8 | Complies |

The acceptance criteria of the test are met, so the test is deemed valid.

*Results*

**Cell viability**

| Sample | 72h |
|---|---|
| | % cell viability |
| Depigmenting emulsion SPF 20 Batch/: PD003 0.1 mg/ml | *80.68 (0.68)* |
| Depigmenting emulsion SPF 20 Batch/ PD003 0.2 mg/ml | *78.05 (0.35)* |
| Hydroquinone/ 5 $\mu$g/ml | *68.94 (0.76)* |
| Negative control | *100.00 (1.16)* |

**Conclusions**

The emulsion of example 1 reduces expression of the RNA messenger coding for tyrosinase in human melanoma cell cultures compared with the untreated control after 72 hours' treatment.

**Claims**

1. Topical formulations comprising oligopeptide-68, encapsulated in vesicular structures selected from liposomes, niosomes, transfersomes and ethosomes, N-acetylglucosamine and at least one UVA sunscreen, optionally combined with one or more UVB sunscreens.

2. Formulations according to claim 1 wherein the oligopeptide-68 is encapsulated in liposomes.

3. Formulations according to claim 1 or 2 wherein the percentage of each UVA or UVB sunscreen ranges from 2.5 to 12.5% by weight.

4. Formulations according to claim 3 wherein the sunscreens are selected from ethylhexyl methoxycinnamate (INCI: Ethylhexyl methoxycinnamate), butyl methoxydibenzoylmethane (INCI: Butyl Methoxydibenzoylmethane), octocrylene (INCI: Octocrylene), salicylic acid derivatives (INCI: EthylhexylSalicylate) and benzophenones (INCI: Benzophenone-3, Benzophenone-4), and mixtures thereof.

5. Formulations according to any one of claims 1 to 4 wherein oligopeptide-68 is present in a range from 0.01 to 10% by weight of the finished preparation.

6. Formulations according to any one of claims 1 to 5 wherein N-acetylglucosamine is present in a range from 0.01 to 10% by weight of the finished preparation.

7. Formulations according to any one of claims 1 to 6 in the form of hydrogels, lipogels, O/W emulsions, W/O emulsions, oils, foundation, BB creams, concealers, cosmetic pencils, leave-on and peel-off masks.

8. Formulations according to any one of claims 1 to 7 further comprising a melanic depigmenting agent selected from diacetyl boldine (INCI: Diacetyl boldine), azelaic acid (INCI: Azelaic acid), dipotassium azeloyl diglycinate (INCI: Dipotassium Azeolyl Diglycinate), sodium ascorbyl phosphate (INCI: Sodium Ascorbyl Phosphate), ethyl ascorbic acid (INCI: Ethyl Ascorbic Acid), ascorbyl hyaluronate (INCI: Ascorbyl hyaluronate), soybean oil (Lecithin), arbutin (INCI: Arbutin), hexyl resorcinol (INCI: hexyl resorcinol) and acetylglycyl alanine (INCI: Acetyl Glycyl $\beta$-Alanine), in amounts ranging from 0.01 to 10% by weight of the finished preparation.

9. The formulations of claims 1-8 in the form of cosmetic products, medical devices or proprietary medicaments.

10. The formulations of claims 1-8 for use in the treatment of cases of acquired melanosis, post-inflammatory melanosis, UV-induced melanosis and photoaging which are resistant to treatment with common depigmenting substances.

Europäisches
Patentamt

European
Patent Office

Office européen
des brevets

# EUROPEAN SEARCH REPORT

Application Number

EP 21 17 7818

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE GNPD [Online] MINTEL; 29 February 2012 (2012-02-29), anonymous: "Resurfacing Eye Serum", XP055514128, Database accession no. 1746797 * abstract * | 1-10 | INV. A61K8/35 A61K8/37 A61K8/40 A61K8/60 A61K8/64 A61Q17/04 A61Q19/02 A61Q19/08 |
| Y | PRATCHYAPURIT WALAI-ORN: "Combined use of two formulations containing diacetyl boldine, TGF-[beta]1 biomimetic oligopeptide-68 with other hypopigmenting/exfoliating agents and sunscreen provides effective and convenient treatment for facial melasma. Either is equal to or is better than", JOURNAL OF COSMETIC DERMATOLOGY, vol. 15, no. 2, 1 June 2016 (2016-06-01), pages 131-144, XP055777014, GB ISSN: 1473-2130, DOI: 10.1111/jocd.12201 * left column; page 133 * | 1-10 | |
| Y | US 2019/076346 A1 (ARMSTRONG ERNEST T [US]) 14 March 2019 (2019-03-14) * claim * * * paragraph [0100] * | 1-10 | TECHNICAL FIELDS SEARCHED (IPC) A61K A61Q |
| Y | LIMA TAMYRES NASSA ET AL: "Bioactive Peptides: Applications and Relevance for Cosmeceuticals", COSMETICS, vol. 5, no. 21, 5 March 2018 (2018-03-05), pages 1-9, XP055776893, DOI: 10.3390/cosmetics5010021 * page 3 * | 1-10 | |

The present search report has been drawn up for all claims

| Place of search | Date of completion of the search | Examiner |
|---|---|---|
| Munich | 8 September 2021 | Grenouillat, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 17 7818

This annex lists the patent family members relating to the patent documents cited in the above-mentioned European search report.
The members are as contained in the European Patent Office EDP file on
The European Patent Office is in no way liable for these particulars which are merely given for the purpose of information.

08-09-2021

| Patent document cited in search report | Publication date | Patent family member(s) | Publication date |
|---|---|---|---|
| US 2019076346 A1 | 14-03-2019 | AU 2017208721 A1 | 09-08-2018 |
| | | BR 112018015015 A2 | 18-12-2018 |
| | | CA 3012082 A1 | 27-07-2017 |
| | | CN 108883316 A | 23-11-2018 |
| | | EP 3405264 A1 | 28-11-2018 |
| | | JP 2019502745 A | 31-01-2019 |
| | | KR 20190008831 A | 25-01-2019 |
| | | US 2019076346 A1 | 14-03-2019 |
| | | WO 2017124156 A1 | 27-07-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MENKE HE et al.** *Ned Tijdschr Geneesk,* 1992, vol. 4, 136
- **BRUCE S et al.** *J. Am. Acad. Dermatol.,* August 1986, vol. 15 (2), 357-61
- **FINDLAY GH.** *S. Afr. Med. J.,* 1980, vol. 57, 187